# EUROPEAN PATENT SPECIFICATION

(11) **EP 1 062 911 B1**
(45) Date of publication and mention of the grant of the patent: **20.06.2007**
(21) Application number: 00113050.9
(22) Date of filing: 23.06.2000
(51) Int. Cl.: A61B 19/00, A61B 6/12, A61B 5/055, G01R 33/28, A61B 6/00, G01R 33/58

(54) **A marker for computer tomography or MRI imaging**
Marker für Computertomografie und MRI-Bilderzeugung
Marqueur pour tomographie à ordinateur et imagerie par RMN

(30) Priority: 24.06.1999 JP 17774099
(43) Date of publication of application: 27.12.2000
(73) Proprietor: Alcare Co., Ltd., Sumida-ku, Tokyo (JP)
(72) Inventor: Hirano, Hiroyuki, Funabashi-shi, Chiba-ken (JP); Takekawa, Naomitsu, Itabashi-ku, Tokyo (JP)
(74) Representative: Klingseisen, Franz

(56) References cited:
- WO-A-00/38579
- US-A- 4 916 170
- US-A- 4 951 673
- US-A- 5 427 099
- PATENT ABSTRACTS OF JAPAN vol. 1998, no. 11, 30 September 1998 (1998-09-30) & JP 10 165391 A (JAPAN FOCUS KK;SONDERLEGER MARSEL), 23 June 1998 (1998-06-23)

## Description

### BACKGROUND OF THE INVENTION

### Field of the Invention

The present invention relates to a marker for image photographing to be used in CT photographing and MRI photographing.

### Background Information

In the medical treatment for focuses such as tumors and so forth in patients brains and bodies, widely conducted is radiotherapy, where radioactive ray is irradiated from outside of the body. In this radiotherapy, it is effective to minimize irradiation amount onto normal tissues (cells) while to irradiate a large amount of radioactive ray onto focuses concerned. For this purpose, it is necessary to irradiate a small exposure dose of radioactive ray onto focuses precisely from multiple directions.

In practice of such radiotherapy, it is required to make a medical treatment plan on irradiation positions, irradiation directions, exposure dose, exposure times and so forth. For making such a plan, it is indispensable to make out the size of a focus concerned in the body and the 3-dimensional position thereof in a precise manner.

In such medical treatment plans, at present, Computed Tomography (CT) photographing using radioactive ray is carried out to investigate the size and position of a focus, and in photographing, a marker is used, and changes in positions of marker and focus displayed on images of laminagram are read and reconfigured to specify the position and so on of a focus concerned.

Conventionally, as such CT marker, a metallic wire has been employed from characteristics of X-ray absorption, it is true that this conventional marker appears clearly on CT images. However, as a wire may damage portions of patient, thus it has been dangerous. It will rust in long term service, and accordingly it has poor durability, and has high fear of contamination, as a result, metallic wire as CT marker has been far from satisfaction in the art.

In practice of the above medical treatment plans, also conducted is Magnetic Resonance Imaging (MRI) photographing, and in this case too, results of tomography using marker are reconfigured in the same manner as stated above so as to specify the size and position of a focus concerned.

Generally, in MRI there is not so significant difference between the proton density of normal tissues and that of focuses, while there is a significant difference between their respective relaxation time. This relaxation time has two factors, i.e., longitudinal relaxation (T1) and transverse relaxation (T2), T1 and T2 in general have nature to offset signal strength, consequently, it is necessary to separately photograph images where T1 is emphasized (T1-weighted spin-echo images) and images where T2 is emphasized (T2-weighted spin-echo images).

Conventionally as marker for this T1-weighted spin-echo images, margarine, salad oil, water solutions of paramagnetic materials and so forth have been employed, while water has been employed as marker for T2-weighted spin-echo images. The markers must be changed prior to respective cases of photographing T1-weighted spin-echo images and T2-weighted spin-echo images, which in turn leads to deteriorated precision in positioning a focus concerned, and further makes handling markers and photographing complicated and troublesome. These have been the problems with the conventional art in the present field.

Accordingly, we formerly provided markers wherein paramagnetic material was added uniformly into viscous liquid and gel hydrophilic substance, which enabled to photograph both T1- and T2-weighted spin-echo images. Furthermore, the present invention aims at making them easier to use, and give them more excellent durability.

Furthermore, US patent No. 4,916,170 discloses a process for making a skin marker, which enables CT image photographing and MRI image photographing to be performed using a single marker. In this conventional process, a hydrogel containing a substantial quantity of water is filled in a plastic tube in which is dispersed a nonmagnetic and X-ray radioopaque material such as barium sulfate.

### SUMMARY OF THE INVENTION

It is an object of the present invention to obtain a marker that can be employed for both CT and MRI, which enables to conduct CT image photographing and MRI (T1, T2) image photographing by use of a single marker and shows more excellent durability.

The present invention has been completed on the basis of the knowledge that we found in our search for a new CT marker to replace the conventional metallic wire, that silicon resin and fluorocarbon resin appeared clearly on CT images.

And as a marker for MRI, we found that elastomer of rubber and so forth, and organogel of polyethylene gel and so forth output high signals in response in photographing T1 and T2-weighted spin-echo images, and appear clearly on images, thereby obtaining an MRI marker having excellent durability.

Still further, a combination of the above CT marker and MRI marker enables to obtain clear images in photographing all the CT, T1 and T2 in MRI images without mutual image interference. Accordingly it enables a single marker to be employed in both the above CT and MRI image photographing operations.

### DETAILED DESCRIPTION OF THE PREFERRED EMBODIMENTS

In photographing CT images, CT parameters are generally employed, and these CT parameters are determined by the absorption dose of X-ray used, so the values vary from -1000 to +1000, wherein CT parameter for air is -1000, that for water is 0, and that for a matter which absorbs all is defined as +1000. In general, CT parameter of bone varies from +300 to +1000, and that of soft tissues is around -80 to -50, while that of brain or liver as objects in normal diagnosis appears from +35 to +100, and that of lungs appears from -800 to -700, as a consequence, it is preferable for a marker to have CT parameter at least over 0, and preferably over 60.

The CT parameter of the silicon resin mentioned above is around from +130 to +230, and that of the fluoroethylene resin mentioned above appears nearly same, therefore, they can show clear images in photographing CT images. In addition, these two resins may be employed together at the same time at necessity.

It is preferable that these CT markers are normally employed in the form of solid bars with diameter about 1 ~ 6 mm, preferably about 2 - 4 mm, and the shape of such bars in specified length enables to insert them into insertion hole arranged in a brain stereotactic radiosurgery, brain stereotactic mask, and other diagnostic patient restrainers and to easily position them. Further they will not damage patients, and will not rust, therefore, they have excellent durability too. Moreover, these CT markers may be formed into hollow bars.

In MRI photographing, as a marker to respond to both the above T1-weighted spin-echo images and T2-weighted spin-echo images, elastomers are available in single or in combination: elastomers of styrene isoprene styrene copolymer (SIS), styrene ethylene butylene styrene copolymer (SEBS), styrene ethylene propylene styrene copolymer (SEPS), isoprene rubber (IR), isobutylene isoprene rubber (IIR), natural rubber (NR) and so forth. And as organogel, available in single or in combination are organogel of polyethylene gel, acrylic gel, urethane gel and so forth. The above elastomer and organogel may be used in combination at necessity.

The above polyethylene gel is one wherein a plasticizer is mixed into ethylene styrene copolymer. As plasticizer are employed liquid paraffin, mineral oil, phthalic acid plasticizers such as butyl benzyl phthalate (BBP), di-octyle phthalate (DOP), di-butyl phthalate (DBP), di-iso nonyl phthalate (DINP) and so forth, soy bean oil, and fatty acids such as oleic acid, linoleic acid and so forth.

The above acrylic gel is firstly copolymerized by such main compounds as 2-ethyl hexyl acrylate, butyl acrylate, methyl acrylate, methyl methacrylate and so forth with acrylic acid, methacrylic acid, vinyl acetate and so forth. And plasticizer are added thereto, and crosslinked by chemical compounds such as isocyanate, aziridine, epoxy system and so forth. As plasticizers are used: those similar to those employed in the above polyethylene gel.

The above urethane gel is a gel of urethane resin plasticized by phthalic acid type plasticizer employed in the above polyethylene gel, or a gel of urethane resin polymerized by polyisocyanate and polyole with molecular weight above about 3000.

As these, taken up are, for example, Cosmogel (polyethylene gel of ethylene styrene copolymer plasticized by liquid paraffin, manufactured by Cosmo Instruments Co., Ltd.), Saivinol AT-304 (acrylic gel, manufactured by Saiden Chemical Industry Co., Ltd.), and so forth.

The elastomer and organogel is filled into a tube or contained into bar shape by a sheet. The tube or sheet is formed of materials that do not affect on T1 and T2-weighted spin-echo images of MRI, for instance, nylon, polyester, polypropylene, polyvinyl chloride and so forth. The tube or bar having elastomer and organogel may be used conveniently in the same manner as stated in the above CT image photographing. And they may be formed into bar shape or so by use of mold.

While the inventors tried combinations of elastomers and organogels that may be used as the above MRI marker, and those that may be employed as the above CT marker, the inventors found that above combination attained appropriate X-ray absorption in CT photographing, and they output high signals in both T1 and T2 of MRI, thus they do not interfere with each other in both CT photographing and MRI photographing.

Namely, the combination marker has sufficient sensivity as a marker for both CT photographing and MRI photographing when the material for the above MRI marker is filled into a hollow tube formed of a material that may be employed as the above CT marker.

In this case, the material for MRI marker may be contained by the material for the above CT marker, or both the materials may be laminated, or formed into bar shapes and so forth.

In the CT photographing mentioned above, it is easy to make out the position of a focus concerned but difficult to make out the shape thereof, while in MRI photographing, wherein there is image distortion peculiar to MRI, it is easy to make out the shape and size of a focus concerned, but difficult to make out the position thereof. The use of the above marker that responds to both above CT photographing and MRI photographing enables to carry out CT photographing with this marker fixed onto a patient, and then carry out MRI photographing without replacing this marker with other. Accordingly, it is possible to prevent displacement in both the CT and MRI images, and to make out the position, shape, and size of a focus concerned in a more precise manner.

And in these years, the development of open magnetic resonance imaging and the progress of computer software have enabled to obtain images wherein distortion of MRI images are revised. In creating synthetic images from these MRI images and CT images, the marker according to the present invention may greatly help increase precision more, and make more appropriate medical treatment plans.

The invention is illustrated in more details by reference to the following referential examples and preferred embodiments hereunder.

### EXAMPLE 1

A marker of solid silicon cylindrical bar shape with diameter 2 mm and length 10 cm was prepared.

CT photographing was conducted by use of this marker, and it has been found that this marker has sufficient absorption and consequently appears clearly on CT images, which shows it is useful as a CT marker.

### EXAMPLE 2

Cosmogel IC00N (polyethylene gel manufactured by Cosmo Instruments Co., Ltd.) was heated up to 130°C and melt, and then suctioned and filled into a polypropylene tube with inner diameter 2 mm and outer diameter 3 mm, and then cooled down to prepare a marker with length 10 cm.

MRI photographing was carried out by use of this marker, and it has been found that this marker outputs high signals to both T1-weighted spin-echo images and T2- - weighted spin-echo images and accordingly appears clearly on both the images, which shows it is useful as an MRI marker.

### EXAMPLE 3

Cosmogel IC00N was heated up to 130°C and melt, and then suctioned and filled into a silicon tube with inner diameter 2 mm and outer diameter 3 mm, and then cooled down to prepare a marker with length 10 cm.

CT photographing was carried out by use of this marker, and it has been found that this marker has sufficient absorption and consequently appears clearly on CT images. And also MRI photographing was carried out by use of this marker, as a result this marker output high signals to both T1-weighted spin-echo images and T2-weighted spin-echo images and accordingly appeared clearly on both the images. This single marker is useful for both CT and MRI (T1, T2) marker.

### EXAMPLE 4

Saivinol AT-304 (acrylic gel manufactured by Saiden Chemical Industry Co., Ltd.) was applied onto a released paper. And its solvent was volatilized, thereby dry acrylic gel of thickness 500µm was manufactured, and then a silicon sheet of thickness 1mm was laminated onto the above dry acrylic gel. And the released paper of acrylic gel was peeled off, and further a silicon sheet of thickness 1mm was attached onto the acrylic gel. Then the acrylic gel was sandwiched by silicon sheets, and was cut, thereby a bar shaped marker with width 3 mm and length 10 cm was made.

As a result, this marker output high signals to all of CT images by CT photographing, T1-weighted spin-echo images and T2-weighted spin-echo images by MRI photographing and appeared clearly on each of those images, thus it has been found that this marker is useful as both CT and MRI marker.

As mentioned heretofore, CT marker according to the present invention shows appropriate X-ray absorption in CT photographing and appears clearly on images. This does not have such fear of damage on patients as the conventional metallic wire does, therefore, it is feasible to obtain a marker that may be used in a safer manner for a long period.

And further MRI marker according to the present invention outputs high signals in both T1-weighted spin-echo images and T2-weighted spin-echo images in MRI photographing and accordingly appears clearly on images. It has excellent storage property and durability since it is made of elastomer and organogel.

Moreover, a combination of the above CT marker and MRI marker appears clearly on images without interfering with each other in both CT photographing and MRI photographing, and still further there is no need to exchange markers for each of these photographing operations, accordingly, it is possible to prevent displacement in both the CT and MRI images. Consequently it may be easily understood by those skilled in the art that it is feasible to obtain images marked with high precision, thereby it is feasible to make more appropriate medical treatment plans.

The invention may be embodied in other specific forms. The present embodiments are therefore to be considered as illustrative, the scope of the invention being indicated by the appended claims.

## Claims

1. A CT/MRI marker for CT and MRI image photographing, **characterized by** that part or whole of a circumference of a bar of an organogel is covered with at least one of a silicon resin and/or a fluorocarbon resin.

2. A CT/MRI marker for CT and MRI image photographing according to claim 1, wherein the organogel comprises at least one of polyethylene gel, acrylic gel and urethane gel.

3. A CT/MRI marker for CT and MRI image photographing according to claim 2, wherein the polyethylene gel comprises a plasticizer mixed into ethylene styrene copolymer.

4. A CT/MRI marker for CT and MRI image photographing according to claim 3, wherein the plasticizer comprises one of liquid paraffin, mineral oil, a phthalic acid plasticizer, soy bean oil and a fatty acid.

5. A CT/MRI marker for CT and MRI image photographing according to claim 4, wherein the phthalic acid plasticizer comprises one of butyl benzyl phthalate (BBP), di-octyle phthalate (DOP), di-butyl phthalate (DBP), and di-iso nonyl phthalate (DINP).

6. A CT/MRI marker for CT and MRI image photographing according to claim 4, wherein the fatty acid comprises one of oleic acid and linoleic acid.

7. A CT/MRI marker for CT and MRI image photographing according to claim 2, wherein the acrylic gel is copolymerized by a main component comprising one of 2-ethyl hexyl acrylate, methyl acrylate, methyl methacrylate and butyl acrylate, and one of acrylic acid, methacrylic acid and vinyl acetate.

8. A CT/MRI marker for CT and MRI image photographing according to claim 7, further comprising a plasticizer and crosslinked by a chemical compound selected from the group consisting of isocyanate, aziridine and an epoxy.

9. A CT/MRI marker for CT and MRI image photographing according to claim 2, wherein the urethane gel comprises one of a gel of a urethane resin plasticized by a phthalic acid type plasticizer and a gel of urethane resin polymerized by polyisoeyanate and polyol with a molecular weight above about 3000.

## Patentansprüche

1. CT/MRI-Marker für CT- und MRI-Bildaufnahme, **dadurch gekennzeichnet, dass** ein Teil oder der gesamte Umfang eines Stabes eines Organogels mit wenigstens einem eines Siliziumharzes und/oder eines Fluorkohlenstoffharzes überzogen ist.

2. CT/MRI-Marker für CT- und MRI-Bildaufnahme nach Anspruch 1, bei welchem das Organogel wenigstens eine Komponente aus Polyethylengel, Acrylgel und Urethangel aufweist.

3. CT/MRI-Marker für CT- und MRI-Bildaufnahme nach Anspruch 2, bei welchem das Polyethylengel einen in ein Ethylenstyrolcopolymer gemischten Weichmacher aufweist.

4. CT/MRI-Marker für CT- und MRI-Bildaufnahme nach Anspruch 3, bei welchem der Weichmacher eine Komponente aus flüssigem Paraffin, Mineralöl, Phtalsäure-Weichmacher, Sojabohnenöl und Fettsäure aufweist.

5. CT/MRI-Marker für CT- und MRI-Bildaufnahme nach Anspruch 4, bei welchem der Phtalsäure-Weichmacher eine Komponente aus Butylbenzylphtalat (BBP), Di-Octylphtalat (DOP), Di-Butylphtalat (DPB) und Di-Iso-Nonylphtalat (DINP) aufweist.

6. CT/MRI-Marker für CT- und MRI-Bildaufnahme nach Anspruch 4, bei welchem die Fettsäure eine Komponente aus Ölsäure und Leinölsäure aufweist.

7. CT/MRI-Marker für CT- und MRI-Bildaufnahme nach Anspruch 2, bei welchem das Acrylgel durch eine Hauptkomponente einer Komponente aus 2-Ethylhexylacrylat, Methylacrylat, Methylmethacrylat und Butylacrylat und einer Komponente aus Acrylsäure, Methacrylsäure und Vinylacetat copolymerisiert ist.

8. CT/MRI-Marker für CT- und MRI-Bildaufnahme nach Anspruch 7, ferner mit einem Weichmacher und quervernetzt durch eine chemische Verbindung, ausgewählt aus der Gruppe bestehend aus Isocyanat, Aziridin und einem Epoxid.

9. CT/MRI-Marker für CT- und MRI-Bildaufnahme nach Anspruch 2, bei welchem das Urethangel eine Komponente eines Gels eines Urethanharzes, das durch einen Weichmacher des Phtalsäuretyps vorgeweicht ist, und eines Gels aus Urethanharz, das durch Polyisocyanat und Polyol mit einem Molekulargewicht über etwa 3000 polymerisiert ist, aufweist.

## Revendications

1. Un marqueur pour CT/IRM pour la photographie des images fournies par tomographie par ordinateur et imagerie résonance magnétique, **caractérisé en ce qu'**une pièce ou l'entièreté d'une circonférence d'une barre d'un organogel est couverte d'une résine silicone ou d'une résine fluorocarbone ou de toutes les deux.

2. Un marqueur pour CT/IRM pour la photographie des images fournies par tomographie par ordinateur et imagerie résonance magnétique selon la revendication 1, dans laquelle l'organogel comprend un gel polyéthylène et/ou un gel acrylique et/ou un gel d'uréthane.

3. Un marqueur pour CT/IRM pour la photographie des images fournies par tomographie par ordinateur et imagerie résonance magnétique selon la revendication 2, dans laquelle le gel polyéthylène comprend un plastifiant mêlé dans un copolymère éthylène-styrène.

4. Un marqueur pour CT/IRM pour la photographie des images fournies par tomographie par ordinateur et imagerie résonance magnétique selon la revendication 3, dans laquelle le plastifiant comprend une paraffine liquide ou une huile minérale ou un plastifiant d'acide phthalique ou de l'huile de grains de soja ou un acide gras.

5. Un marqueur pour CT/IRM pour la photographie des images fournies par tomographie par ordinateur et imagerie résonance magnétique selon la revendication 4, dans laquelle le plastifiant d'acide phthalique comprend de butyl-benzyl-phthalate (BBP), de dioctyl phthalate (DOP), de di-butyl phthalate (DiBP) ou de di-iso-nonyl phthalate (DINP).

6. Un marqueur pour CT/IRM pour la photographie des images fournies par tomographie par ordinateur et imagerie résonance magnétique selon la revendication 4, dans laquelle l'acide gras comprend d'acide oléique ou d'acide linoléique.

7. Un marqueur pour CT/IRM pour la photographie des images fournies par tomographie par ordinateur et imagerie résonance magnétique selon la revendication 2, dans laquelle le gel acrylique est copolymérisé par un élément principal comprenant l'un de di-éthylhexyl acrylate, d'acrylate de méthyle, de méthacrylate de méthyle, ou d'acrylate de butyle, et l'un d'acide acrylique, d'acide méthacrylique ou d'acétate de vinyle.

8. Un marqueur pour CT/IRM pour la photographie des images fournies par tomographie par ordinateur et imagerie résonance magnétique selon la revendication 7, comprenant de plus un plastifiant et lié transversale par une combinaison chimique choisie du groupe comportant de l'isocyanate, de l'aziridine et une époxy.

9. Un marqueur pour CT/IRM pour la photographie des images fournies par tomographie par ordinateur et imagerie résonance magnétique selon la revendication 2, dans laquelle le gel d'uréthane comprend un gel d'une résine uréthane plastifié par un plastifiant du type d'acide phthalique ou un gel de résine uréthane polymérisé par du polyisocyanate et du polyol avec un poids moléculaire au-dessus d'environ 3000.
